# EUROPEAN PATENT APPLICATION

(11) **EP 4 814 132 A1**
(43) Date of publication of application: **30.09.2026**
(21) Application number: 24894630.3
(22) Date of filing: 21.11.2024
(51) Int. Cl.: C12Q 1/6886

(54) **COMPOSITION FOR DETECTING PIK3CA GENE MUTANT TUMOR IN COMPANION ANIMAL AND METHOD FOR DIAGNOSING PIK3CA GENE MUTANT TUMOR IN COMPANION ANIMAL USING SAME**

(30) Priority: 24.11.2023 KR 20230165966; 12.11.2024 KR 20240160246
(71) Applicant: Canicaticare, Inc., Daegu 42078 (KR)
(72) Inventor: HONG, Jae Woo, Daegu 42171 (KR); ZHU, An Lin, Daegu 42459 (KR)
(74) Representative: Wichmann, Hendrik
(86) International application number: PCT/KR2024/018465
(87) International publication number: WO 2025/110738

(57) **Abstract**

The present disclosure relates to a primer set for detecting a PIK3CA gene mutant tumor in a companion animal, the primer set including a forward primer selected from the group consisting of primers represented by SEQ ID NOs: 1 to 3 and a reverse primer selected from the group consisting of primers represented by SEQ ID NOs: 4 to 8.

## Description

### [Technical Field]

The present disclosure relates to a composition for detecting a PIK3CA gene mutant tumor in a companion animal including a primer set capable of amplifying a PIK3CA gene mutation in a companion animal, and a method for diagnosing a PIK3CA gene mutant tumor in a companion animal using the same. The present disclosure provides information necessary for diagnosis and treatment of a PIK3CA gene mutant tumor by detecting a PIK3CA gene mutation in a companion animal.

### [Background Art]

As it has become known that mutations in oncogenes and tumor suppressor genes are involved in the occurrence of cancer in animals including humans, various studies have been conducted to detect mutations in various oncogenes and tumor suppressor genes. The detection of these mutations is involved in determining the occurrence of cancer and the prognosis of drugs to provide information necessary for the prevention, diagnosis, or treatment of cancers or tumors.

The mutations of a PIK3CA gene cause the activation of a phosphatidylinositol 3-kinase (PI3K) protein, and the PI3K activates a signaling system of a tyrosine kinase receptor. The activated tyrosine kinase is involved in a carcinogenesis process by activating a protein function of inhibiting cell survival and division and a protein function of interfering with a metabolism and assisting cell growth. The PIK3CA gene mutations accelerate cancer progression and increase the frequency of metastasis to worsen the prognosis of a patient. It is also known to be involved in resistance to targeted therapeutic agents of the HER family, which belongs to the downstream signaling system of a human epidermal growth factor (HER family).

Unlike in humans, diseases such as tumors in animals have no auxiliary diagnostic or detection means, such as a detection test of appropriate biomarkers for tumor-related diseases, including regular checkups, and the like. In the case of animals, regular health checkups are not performed, and checkups are conducted too late when signs of the disease appear, thereby making prevention of the disease difficult. In addition, there are limitations because animals may not usually undergo precise examinations, such as contrast inspection and tissue biopsy, at general animal hospitals. In general, in the case of humans, when biomarkers are used as indicators that may detect changes within the body by using proteins, DNA, RNA (ribonucleic acid), metabolites, etc., normal or pathological conditions of living organisms, the degree of response to drugs, and the like may be objectively measured.

Under this background, the present inventors completed the present disclosure to provide information necessary for the diagnosis of related diseases by detecting a PIK3CA gene mutation in a companion animal to diagnose changes within the body of the companion animal and preparing a primer set for detecting a PIK3CA gene mutant tumor.

### [Prior Arts]

### [Patent Documents]

Korean Patent Registration No. 10-1543215

### [Disclosure]

### [Technical Problem]

An object of the present disclosure is to provide a method for very simply and rapidly detecting a tumor in a companion animal using a composition for detecting a PIK3CA gene mutant tumor in a companion animal, and performing clinical diagnosis quickly and easily with high reliability.

### [Technical Solution]

An aspect of the present disclosure provides a primer set for detecting a PIK3CA gene mutant tumor in a companion animal, the primer set including a forward primer selected from the group consisting of primers represented by SEQ ID NOs: 1 to 3 and a reverse primer selected from the group consisting of primers represented by SEQ ID NOs: 4 to 8, and a composition for detecting a PIK3CA gene mutant tumor in a companion animal including the same. Another aspect of the present disclosure provides a method for diagnosing a PIK3CA gene mutant tumor in a companion animal using the primer set and information necessary for the treatment of a PIK3CA gene mutant tumor in a companion animal.

### [Advantageous Effects]

According to the present disclosure, it is possible to detect a PIK3CA gene mutant tumor in a companion animal with high accuracy and sensitivity, perform clinical diagnosis quickly and easily with high reliability, and provide information necessary for tumor treatment by performing RT-qPCR using the composition and the method.

### [Description of drawings]

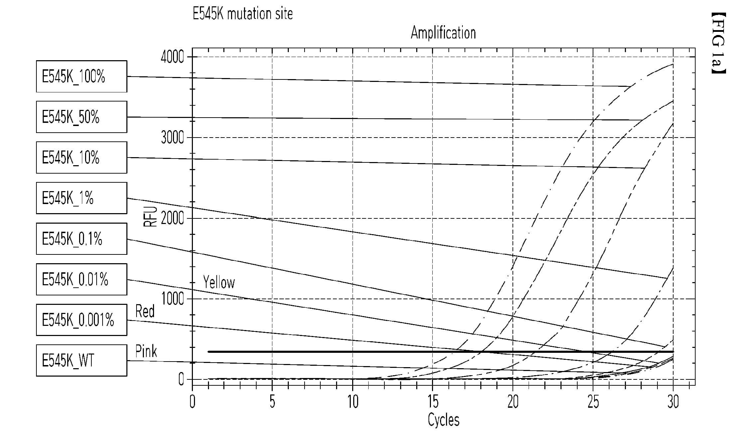

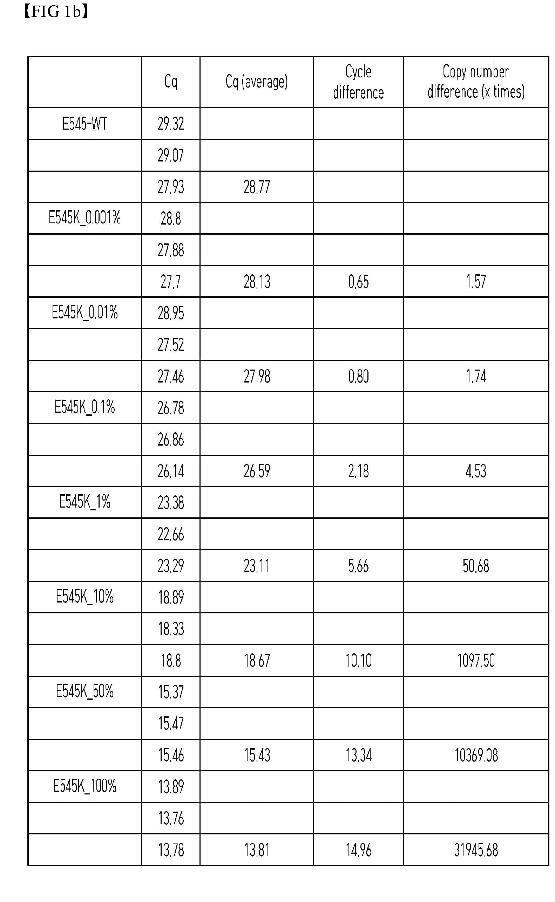

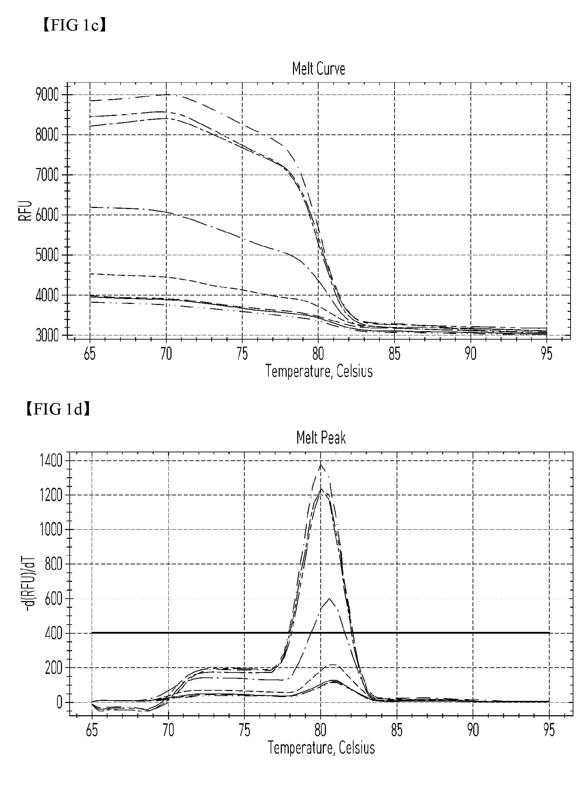

FIG. 1 illustrates a result of performing PCR (RP-qPCR) using a composition for detecting a PIK3CA gene mutant tumor in a companion animal including a primer set according to an embodiment of the present disclosure. FIGS. 1A and 1B illustrate results of confirming PCR amplification and evaluating analytic sensitivity for template DNA of Accession Number: NC_051838 by setting a mutant DNA ratio to 0.001%, 0.01%, 0.1%, 1%, 10%, 50%, and 100%. FIGS. 1A and 1B illustrate results of performing PCR amplification using a composition for detecting a PIK3CA gene mutant tumor in a companion animal including a primer set including at least any one selected from the group consisting of forward primers represented by SEQ ID NOs: 1 and 2 and a reverse primer represented by SEQ ID NO: 4. FIGS. 1C and 1D illustrate results of analyzing gene amplification of RT-qPCR using a melting curve analysis method in a step of detecting a PIK3CA mutation in a companion animal by analyzing the gene amplification of the RT-qPCR.

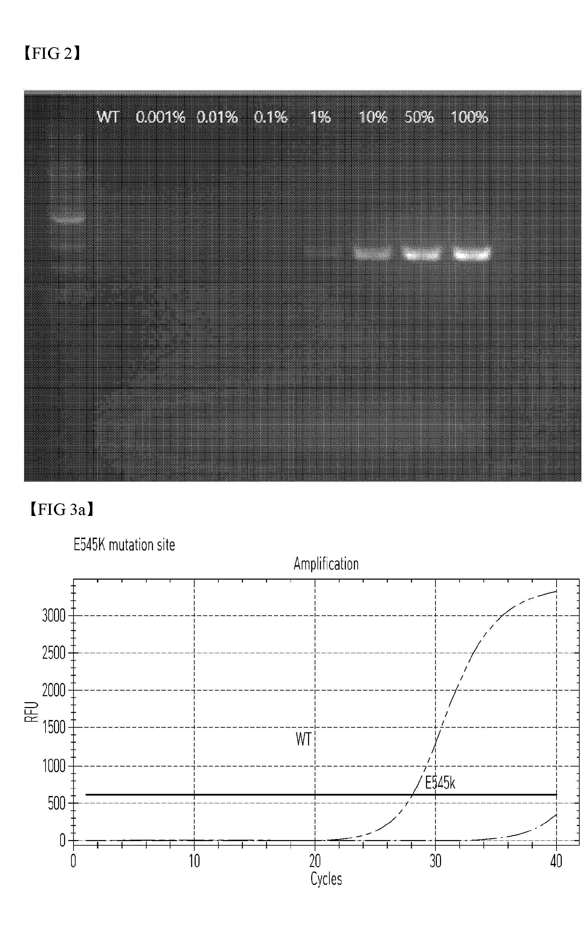

FIG. 2 illustrates a result of performing PCR (RP-qPCR) using a composition for detecting a PIK3CA gene mutant tumor in a companion animal including a primer set according to an embodiment of the present disclosure. FIG. 2 illustrates a result of confirming PCR amplification and evaluating analytic sensitivity for template DNA of Accession Number: NC_051838 by setting a mutant DNA ratio to 0.001%, 0.01%, 0.1%, 1%, 10%, 50%, and 100%. FIG. 2 illustrates a result confirmed by agarose gel electrophoresis in a step of detecting a PIK3CA mutation in a companion animal by analyzing gene amplification of RT-qPCR. FIG. 2 illustrates a result of performing PCR amplification using a composition for detecting a PIK3CA gene mutant tumor in a companion animal including a primer set including at least any one selected from the group consisting of forward primers represented by SEQ ID NOs: 1 and 2 and a reverse primer represented by SEQ ID NO: 4.

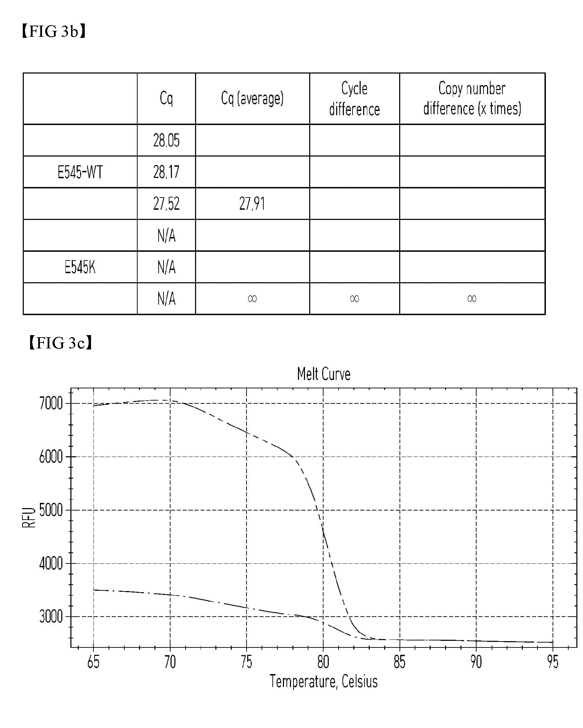

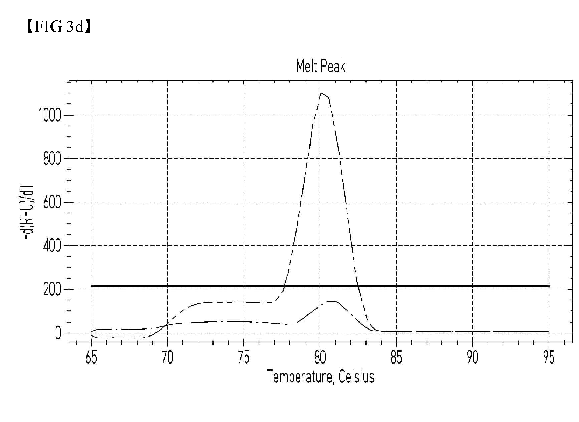

FIG. 3 illustrates a result of performing PCR (RP-qPCR) using a composition for detecting a PIK3CA gene mutant tumor in a companion animal including a primer set according to an embodiment of the present disclosure. FIGS. 3A and 3B illustrate results of performing PCR amplification for template DNA of Accession Number: NC_006616 using a composition for detecting a PIK3CA gene mutant tumor in a companion animal including a primer set including at least any one selected from the group consisting of forward primers represented by SEQ ID NOs: 1 and 2 and a reverse primer represented by SEQ ID NO: 4. FIGS. 3C and 3D illustrate results of analyzing gene amplification of RT-qPCR using a melting curve analysis method in a step of detecting a PIK3CA mutation in a companion animal by analyzing the gene amplification of the RT-qPCR.

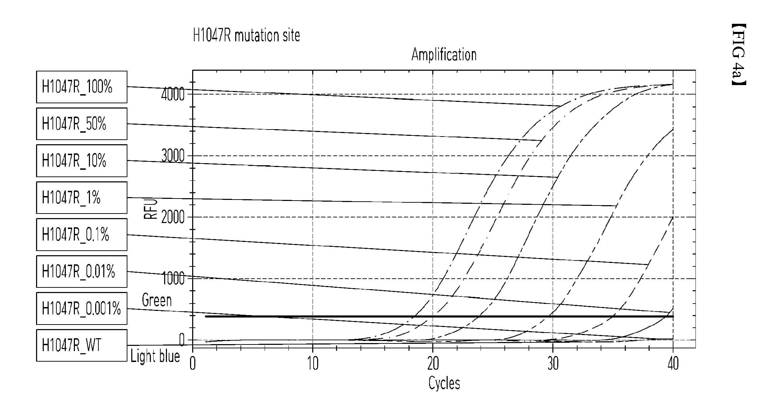

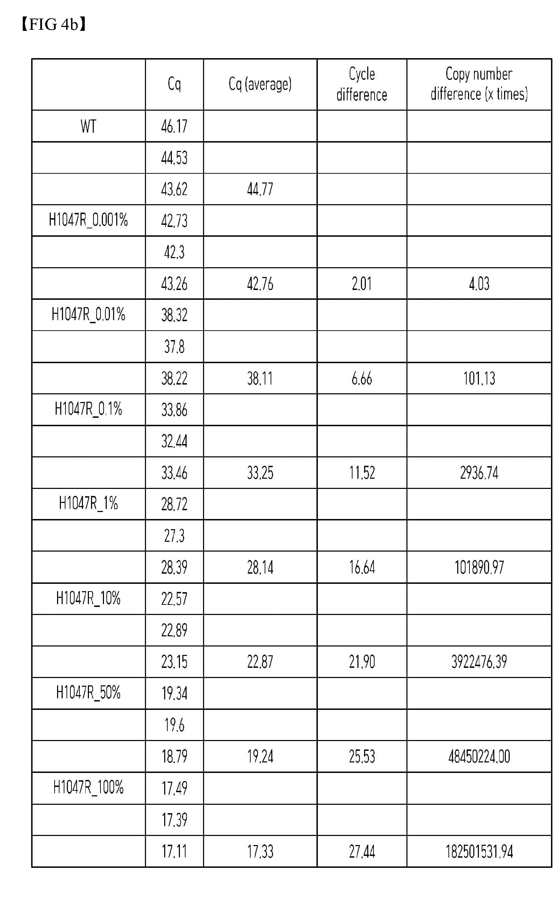

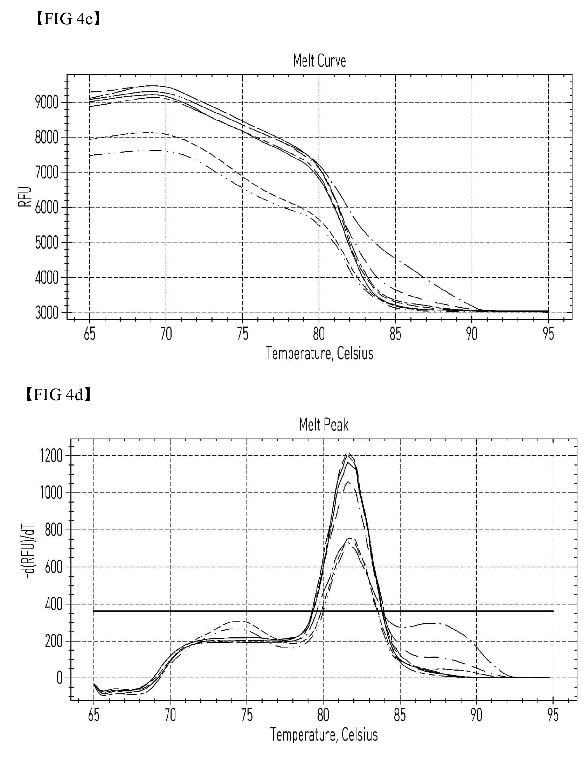

FIG. 4 illustrates a result of performing PCR (RP-qPCR) using a composition for detecting a PIK3CA gene mutant tumor in a companion animal including a primer set according to an embodiment of the present disclosure. FIGS. 4A and 4B illustrate results of confirming PCR amplification and evaluating analytic sensitivity for template DNA of Accession Number: NC_051838 by setting a mutant DNA ratio to 0.001%, 0.01%, 0.1%, 1%, 10%, 50%, and 100%. FIGS. 4A and 4B illustrate results of performing PCR amplification using a composition for detecting a PIK3CA gene mutant tumor in a companion animal including a primer set including at least any one primer selected from the group consisting of a forward primer represented by SEQ ID NO: 3 and reverse primers represented by SEQ ID NOs: 5 to 8. FIGS. 4C and 4D illustrate results of analyzing gene amplification of RT-qPCR using a melting curve analysis method in a step of detecting a PIK3CA mutation in a companion animal by analyzing the gene amplification of RT-qPCR.

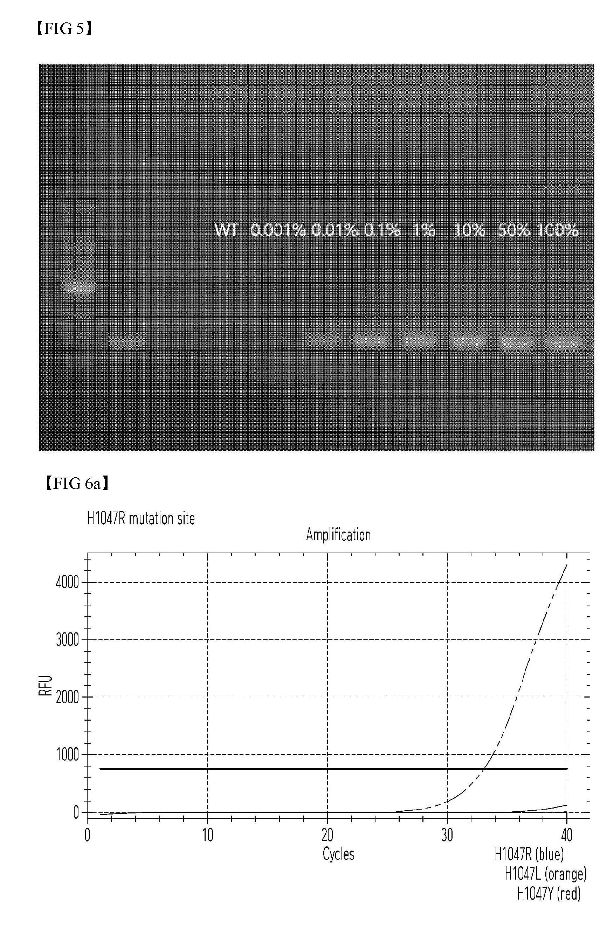

FIG. 5 illustrates a result of performing PCR (RP-qPCR) using a composition for detecting a PIK3CA gene mutant tumor in a companion animal including a primer set according to an embodiment of the present disclosure. FIG. 5 illustrates a result of confirming PCR amplification and evaluating analytic sensitivity for template DNA of Accession Number: NC_051838 by setting a mutant DNA ratio to 0.001%, 0.01%, 0.1%, 1%, 10%, 50%, and 100%. FIG. 5 illustrates a result confirmed by agarose gel electrophoresis in a step of detecting a PIK3CA mutation in a companion animal by analyzing the gene amplification of RT-qPCR. FIG. 5 illustrates a result of performing PCR amplification using a composition for detecting a PIK3CA gene mutant tumor in a companion animal including a primer set including at least any one primer selected from the group consisting of a forward primer represented by SEQ ID NO: 3 and reverse primers represented by SEQ ID NOs: 5 to 8.

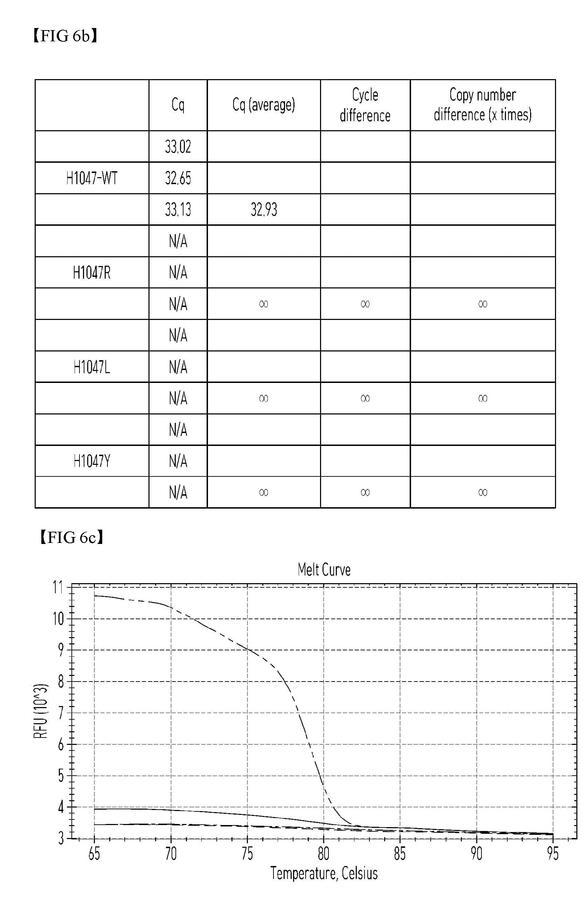

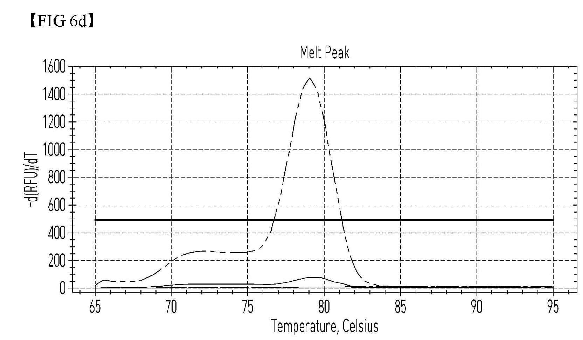

FIG. 6 illustrates a result of performing PCR (RP-qPCR) using a composition for detecting a PIK3CA gene mutant tumor in a companion animal including a primer set according to an embodiment of the present disclosure. FIGS. 6A and 6B illustrate results of performing PCR amplification for template DNA of Accession Number: NC_006616 using a composition for detecting a PIK3CA gene mutant tumor in a companion animal including a primer set including at least any one primer selected from the group consisting of a forward primer represented by SEQ ID NO: 3 and reverse primers represented by SEQ ID NOs: 5 to 8. FIGS. 6C and 6D illustrate results of analyzing gene amplification of RT-qPCR using a melting curve analysis method in a step of detecting a PIK3CA mutation in a companion animal by analyzing the gene amplification of RT-qPCR.

### [Best Mode]

Hereinafter, various embodiments of the present disclosure will be described in detail with reference to the accompanying drawings. The present disclosure is not limited to specific embodiments, and it should be understood to include various modifications, equivalents, and/or alternatives to the embodiments of the present disclosure. In connection with the description of the drawings, similar reference numerals may be used for similar components.

In the present specification, expressions such as "have", "may have", "include", or "may include" refer to the presence of the corresponding feature (e.g., components such as numerical values, functions, operations, or components), and do not exclude the presence of additional features.

In the present specification, the expression such as "A or B", "at least one of A and/or B", or "one or more of A and/or B" may include all possible combinations of items listed together. For example, "A or B", "at least one of A and B", or "at least one of A or B" may refer to all cases of (1) including at least one A, (2) including at least one B, or (3) including both at least one A and at least one B.

The expression of "configured to (or set to)" used herein may be changed and used to, for example, "suitable for", "having the capacity to", "designed to", "adapted to", "made to" or "capable of", depending on the situation. The term "configured to (or set to)" does not necessarily mean only "specially designed to".

The terms used herein are used to illustrate only specific embodiments, and may not be intended to limit the scope of other embodiments. A singular expression may include a plural expression unless the context clearly indicates otherwise. The terms used herein, including technical or scientific terms, may have the same meaning as generally understood by those of ordinary skill in the art described in the present specification. The terms defined in a general dictionary among the terms used herein may be interpreted in the same or similar meaning as or to the meaning in the context of the related art, and will not be interpreted as an ideal or excessively formal meaning unless otherwise defined in the present specification. In some cases, even the terms defined in the present specification cannot be interpreted to exclude the embodiments of the present specification.

The embodiments disclosed in the present specification are presented for explanation and understanding of the disclosed technical contents, and do not limit the scope of the present disclosure. Therefore, the scope of the present specification should be interpreted as including all changes or various other embodiments based on the technical idea of the present disclosure.

Hereinafter, a preferred embodiment of the present disclosure will be described in detail. Terms and words used in the present specification and claims should not be interpreted as being limited to typical or dictionary meanings, but should be interpreted as having meanings and concepts which comply with the technical spirit of the present disclosure, based on the principle that the present inventors can appropriately define the concept of the term to describe their own invention in the best manner.

Therefore, the configurations of the embodiments described in the present specification are merely the most preferred embodiment of the present disclosure and are not intended to represent all of the technical ideas of the present disclosure, and thus, it should be understood that there are various equivalents and modifications capable of replacing the configurations at the time of this application.

Throughout the specification, when a part "includes" a component, unless otherwise specifically stated, it is meant to further include other components rather than excluding other components.

Hereinafter, the present disclosure will be described in detail.

According to an embodiment of the present disclosure, the present disclosure relates to a primer set for detecting a PIK3CA gene mutant tumor in a companion animal including a forward primer selected from the group consisting of primers represented by SEQ ID NOs: 1 to 3 and a reverse primer selected from the group consisting of primers represented by SEQ ID NOs: 4 to 8.

As used herein, the term 'primer' refers to a nucleic acid sequence having a short free 3-terminal hydroxyl group, and a short nucleic acid sequence capable of forming a base pair with a complementary template and serving as a starting point for copying a template strand. The term 'template' refers to a 'template nucleic acid' and means a nucleic acid used as a template for amplification in a PCR reaction. The 'template' may include those that exist in nature, are naturally generated, or are synthetic. For example, in the present disclosure, the 'template' may refer to a PIK3CA gene in a companion animal.

The primer may initiate DNA synthesis in the presence of reagents (DNA polymerase, reverse transcriptase, etc.) for polymerization and four different nucleoside triphosphates in an appropriate buffer solution and temperature. The primer is also referred to as an initiator, and may be in various forms which are formed from DNA, RNA, PNA (peptide nucleic acid), LNA (locked nucleic acid), or mixtures thereof and may be used for gene amplification.

The primer may be prepared through a chemical synthesis method using, for example, a phosphoramidite method, a phosphodiester method, a diethyl phosphoramidite method, etc. In addition, a primer base sequence may be modified by means known in the art. The primer is typically single-stranded oligonucleotide (e.g., oligodeoxyribonucleotide). The length of the primer varies depending on the intended use in the range of consecutive 10 to 50 nucleotides, preferably 15 to 35 nucleotides. A short primer molecule generally requires lower temperatures to form a sufficiently stable hybridization complex with the template. The primer is not required to reflect an exact sequence of the template, but needs to be sufficiently complementary to hybridize with the template for elongation of the primer. The primer may include a label detectable directly or indirectly by spectroscopic, photochemical, biochemical, immunochemical, or chemical means, if necessary. Examples of the label include enzymes (e.g., horseradish peroxidase, alkaline phosphatase), radioactive isotopes (e.g., ³²P), fluorescent molecules, chemical groups (e.g., biotin), etc.

As used herein, the term 'primer set' refers to a plurality of primers. The primer set may further include a container for accommodating the primers. The plurality of primers of the primer set may include forward primers and reverse primers, which refer to general initiators typically used for the amplification of a gene sample containing a gene mutation to be detected. This is a matter that may be easily determined by those skilled in the art related to such a primer set depending on a gene mutation to be detected and a gene containing the mutation.

The primer set of the present disclosure is delicately designed to maximize the sensitivity and specificity of detecting the PIK3CA gene mutation in the companion animal by fully considering a size of the primer, a Tm value, a GC content of the primer, prevention of formation of a primer dimer due to a self-complementary sequence within the primer, prohibition of repeating the same nucleotide sequence three or more times, etc.

As used herein, the term 'mutation' refers to a phenomenon in which a qualitative or quantitative change occurs in a gene due to the abnormality in a gene or chromosome, thereby inducing a change in genetic traits. The mutation may be a mutation at a gene level, and preferably may be a point mutation or multiple mutations selected from substitution, insertion, and deletion. The mutation may be silent mutation, neutral mutation, missense mutation, nonsense mutation, frame shift mutation, etc., and is not limited to these types.

The term 'tumor' used herein may mean a solid tumor. The solid tumor is derived from mesothelioma, cervical cancer, pancreatic cancer, ovarian cancer, squamous cell carcinoma (e.g., epithelial squamous cell carcinoma), small cell lung cancer, non-small cell lung cancer, lung cancer including adenocarcinoma of lung and squamous carcinoma of lung, peritoneal cancer, hepatocellular carcinoma, stomach cancer including gastric cancer, pancreatic cancer, glioblastoma, liver cancer, bladder cancer, hepatoma, breast cancer, colon cancer, rectal cancer, colorectal cancer, endometrial cancer or uterine carcinoma, salivary gland carcinoma, kidney cancer or renal cancer, prostate cancer, vulvar cancer, thyroid cancer, liver carcinoma, anal carcinoma, penile carcinoma, head and neck cancer, or any combination thereof.

The term 'polymerase chain reaction (PCR)' used herein is a method for confirming whether a gene is amplified using a fluorescent substance, and is a gene amplification method capable of simultaneously amplifying and detecting a gene. The PCR reaction may be performed through (i) an initial denaturation step; (ii) a step of repeating one cycle consisting of denaturation, annealing, and extension several to tens of times; and (iii) a final heat treatment step; or a thermal cycle program which is suitably modified with these steps. Specifically, the gene amplification may use one amplification method selected from the group consisting of reverse transcriptase polymerase chain reaction (RT-PCR), real-time reverse transcriptase polymerase chain reaction (real-time RT-PCR), quantitative polymerase chain reaction (qPCR), competitive reverse transcriptase polymerase chain reaction (competitive RT-PCR), real-time quantitative polymerase chain reaction (RT-qPCR), inverse polymerase chain reaction (inverse PCR), and long and accurate polymerase chain reaction (long and accurate PCR). In the present disclosure, the gene amplification using the primer set may preferably use one amplification method selected from the group consisting of RT-PCR, RT-qPCR, and real-time PCR. More preferably, RT-qPCR may be used. 'RT-qPCR' is a combined technology of 'RT-PCR' and 'qPCR' that enables the quantification of gene expression and enables rapid and accurate detection.

PCR, RT-PCR, RT-qPCR, or real-time PCR amplification results may be confirmed using agarose gel electrophoresis, etc. The results of electrophoresis may be analyzed by staining with ethidium bromide after electrophoresis. In the present disclosure, RT-qPCR amplification results may be analyzed using agarose gel electrophoresis or melting curve analysis.

The 'PIK3CA' of the present disclosure is a gene for phosphatidylinositol 3-kinase (PI3K), and the PI3K is a lipid kinase that catalyzes a phosphorylation of phosphatidyl-inositol 4,5-bisphosphate (PIP₂), which is involved in cell signaling, into phosphatidylinositol 3,4,5-trisphosphate (PIP₃). The PI3K is classified into Classes I, II, and III based on structure, mechanism of action, and substrate specificity. Among them, Class IB PI3K contains p110γ, which is activated by a G protein-coupled receptor. A cell signaling pathway mediated by p110α is overactivated in human cancer cells. In addition, when the PI3K cell signaling pathway is abnormally regulated, the PIP3 level is increased to activate a downstream enzyme Akt, and as a result, affects cancer, immune disorders, and cardiovascular disorders. A PIK3CA gene encoding p110α is mutated and overexpressed in various primary cancers. The PIK3CA gene mutation causes the activation of a phosphatidylinositol 3-kinase (PI3K) protein, and the PI3K activates a signaling system of a tyrosine kinase receptor. The activated tyrosine kinase is involved in a carcinogenesis process by activating a protein function that inhibits cell survival and division and a protein function that interferes with a metabolism and helps cell growth. The PIK3CA gene mutation accelerates the cancer progression rate and increases the frequency of metastasis to worsen the prognosis of cancer occurrence. It is also known to be involved in resistance to targeted therapeutic agents of the HER family, which belongs to the downstream signaling system of a human epidermal growth factor (HER family). The PIK3CA gene mutation may cause a tumor, and the tumor may be a mammary tumor. The mammary tumor may be at least one selected from the group consisting of fibroadenoma, adenocarcinoma, and spindle cell neoplasm.

The companion animal of the present disclosure may be at least one selected from the group consisting of companion dogs, cats, mice, rabbits, horses, sheep, hamsters, hedgehogs, ferrets, and guinea pigs. Preferably, the companion animal of the present disclosure may be a companion dog.

In the present disclosure, the primer set for detecting the PIK3CA gene mutant tumor in the companion animal detects one or more mutations selected from the group consisting of PIK3CA_E545K, PIK3CA_E542K, PIK3CA_H1047R, PIK3CA_H1047L, and PIK3CA_H1047Y. The primer set of the present disclosure may respectively amplify any one of PIK3CA_E545K, PIK3CA_E542K, PIK3CA_H1047R, PIK3CA_H1047L, or PIK3CA_H1047Y, or simultaneously amplify one or more mutations selected from the group consisting of PIK3CA_E545K, PIK3CA_E542K, PIK3CA_H1047R, PIK3CA_H1047L, and PIK3CA_H1047Y.

The 'primer set for detecting the PIK3CA gene mutant tumor' of the present disclosure refers to a set of primer pairs capable of specifically binding to one or more mutation sites selected from the group consisting of PIK3CA_E545K, PIK3CA_E542K, PIK3CA_H1047R, PIK3CA_H1047L, and PIK3CA_H1047Y in the PIK3CA gene to induce an elongation reaction. Each of the primer pairs produces a PCR product for the PIK3CA gene mutation, but does not almost produce the PCR product for a wild-type PIK3CA gene.

The primers represented by SEQ ID NOs: 1 to 8 are as follows:
cPIK3CA-E545-WT-fwd-1: 5'- gatcctctctctgaaatcactg-3' (SEQ ID NO: 1)
cPIK3CA-E545K-fwd-1: 5'- gatcctctctctgaaatcacta -3' (SEQ ID NO: 2)
cfPIK3CA-H1047-fwd-2: 5'- catacattcgaaagaccctagc-3' (SEQ ID NO: 3)
cfPIK3CA-E545-rve-1: 5'- aggttagtaccaatgcagcgt-3' (SEQ ID NO: 4)
cPIK3CA-H1047-WT-rve-2: 5'- tgttgtccagccaccatgatg-3' (SEQ ID NO: 5)
cPIK3CA-H1047R-rve-2: 5'- ttgttgtccagccaccatgag-3' (SEQ ID NO: 6)
cPIK3CA-H1047L-rve-2: 5'- ttgttgtccagccaccatgaa-3' (SEQ ID NO: 7)
cPIK3CA-H1047Y-rve-2: 5'- tgttgtccagccaccatgata-3' (SEQ ID NO: 8).

A 1 base at the 3' end of a forward primer represented by SEQ ID NO: 2 may be used for detecting a mutation corresponding to a position having a high mutation possibility in a target DNA sequence.

A 1 base at the 3' end of a reverse primer represented by SEQ ID NO: 6 may be used for detecting a mutation corresponding to a position having a high mutation possibility in a target DNA sequence.

A 1 base at the 3' end of a reverse primer represented by SEQ ID NO: 7 may be used for detecting a mutation corresponding to a position having a high mutation possibility in a target DNA sequence.

A 1 base at the 3' end of a reverse primer represented by SEQ ID NO: 8 may be used for detecting a mutation corresponding to a position having a high mutation possibility in a target DNA sequence.

According to an embodiment of the present disclosure, the present disclosure relates to a composition for detecting a PIK3CA gene mutant tumor in a companion animal including: at least any one primer set selected from the group consisting of a) a primer set consisting of a forward primer represented by SEQ ID NO: 1 and a reverse primer represented by SEQ ID NO: 4; b) a primer set consisting of a forward primer represented by SEQ ID NO: 2 and a reverse primer represented by SEQ ID NO: 4; c) a primer set consisting of a forward primer represented by SEQ ID NO: 3 and a reverse primer represented by SEQ ID NO: 5; d) a primer set consisting of a forward primer represented by SEQ ID NO: 3 and a reverse primer represented by SEQ ID NO: 6; e) a primer set consisting of a forward primer represented by SEQ ID NO: 3 and a reverse primer represented by SEQ ID NO: 7; and f) a primer set consisting of a forward primer represented by SEQ ID NO: 3 and a reverse primer represented by SEQ ID NO: 8.

According to an embodiment of the present disclosure, the present disclosure relates to a composition for detecting a PIK3CA gene mutant tumor in a companion animal including: at least any one primer set selected from the group consisting of a) a primer set consisting of a forward primer represented by SEQ ID NO: 1 and a reverse primer represented by SEQ ID NO: 4; and b) a primer set consisting of a forward primer represented by SEQ ID NO: 2 and a reverse primer represented by SEQ ID NO: 4, in which the primer set detects a PIK3CA_E545K mutation.

According to an embodiment of the present disclosure, the present disclosure relates to a composition for detecting a PIK3CA gene mutant tumor in a companion animal including: at least any one primer set selected from the group consisting of c) a primer set consisting of a forward primer represented by SEQ ID NO: 3 and a reverse primer represented by SEQ ID NO: 5; d) a primer set consisting of a forward primer represented by SEQ ID NO: 3 and a reverse primer represented by SEQ ID NO: 6; e) a primer set consisting of a forward primer represented by SEQ ID NO: 3 and a reverse primer represented by SEQ ID NO: 7; and f) a primer set consisting of a forward primer represented by SEQ ID NO: 3 and a reverse primer represented by SEQ ID NO: 8, in which the primer set detects at least any one mutation selected from the group consisting of PIK3CA_H1047R, PIK3CA_H1047L and PIK3CA_H1047Y.

The composition for detecting the PIK3CA gene mutant tumor in the companion animal including the primer set of the present disclosure may respectively amplify any one of PIK3CA_E545K, PIK3CA_E542K, PIK3CA_H1047R, PIK3CA_H1047L, or PIK3CA_H1047Y, or simultaneously amplify one or more mutations selected from the group consisting of PIK3CA_E545K, PIK3CA_E542K, PIK3CA_H1047R, PIK3CA_H1047L, and PIK3CA_H1047Y.

According to an embodiment of the present disclosure, the present disclosure relates to a method for diagnosing a PIK3CA gene mutant tumor in a companion animal including: performing RT-qPCR for a PIK3CA gene in a companion animal using a primer set for detecting a PIK3CA gene mutant tumor in a companion animal including a forward primer selected from the group consisting of primers represented by SEQ ID NOs: 1 to 3 and a reverse primer selected from the group consisting of primers represented by SEQ ID NOs: 4 to 8; and detecting a PIK3CA mutation in the companion animal by analyzing gene amplification of the RT-qPCR.

The PIK3CA gene mutant tumor in the companion animal may be a mammary tumor. The mammary tumor may be at least one selected from the group consisting of fibroadenoma, adenocarcinoma, and spindle cell neoplasm.

The step of performing the RT-qPCR for the PIK3CA gene in the companion animal using the primer set for detecting the PIK3CA gene mutant tumor in the companion animal including the forward primer selected from the group consisting of primers represented by SEQ ID NOs: 1 to 3 and the reverse primer selected from the group consisting of primers represented by SEQ ID NOs: 4 to 8 means a process of amplifying target DNA by repeating a process of denaturation, annealing, and extension reactions using target DNA as a template and a primer specific to the target DNA. The target DNA may be used as the template using PIK3CA gene information of a dog registered in the NCBI genebank in [Table 1] below.

In the step of performing the RT-qPCR for the PIK3CA gene in the companion animal using the primer set for detecting the PIK3CA gene mutant tumor in the companion animal including the forward primer selected from the group consisting of primers represented by SEQ ID NOs: 1 to 3 and the reverse primer selected from the group consisting of primers represented by SEQ ID NOs: 4 to 8, under specific PCR conditions, PCR amplification may be performed by repeating a total of 10 to 40 times a denaturation step at 90 to 95°C for 10 seconds to 2 minutes; an annealing step at 58 to 70°C for 10 seconds to 2 minutes; and an extension step at 70 to 75°C for 10 seconds to 2 minutes, after initial denaturation by heat treatment at 90 to 95°C for 1 to 20 minutes, and the reaction temperature and reaction time conditions may be appropriately modified and performed by those skilled in the art. More preferably, after initial denaturation at 95°C for 3 minutes, a denaturation step at 95°C for 10 seconds; an annealing step at 66°C for 10 seconds; and an extension step at 72°C for 30 seconds may be performed.

In the step of detecting the PIK3CA mutation in the companion animal by analyzing the gene amplification of the RT-qPCR, the size analysis of a PCR amplification product may be performed by using the method known in the art. Preferably, the size of the amplification product may be confirmed by comparison with a target size marker by agarose or polyacrylamide gel electrophoresis, or the PIK3CA mutation may be detected by melting curve analysis.

The melting curve analysis refers specifically to detecting the PIK3CA gene mutation in the companion animal by confirming a temperature at which double-stranded DNA is dissociated into single-stranded DNA. In the step of detecting the PIK3CA mutation in the companion animal by analyzing the gene amplification of the RT-qPCR, the gene amplification of the RT-qPCR was analyzed using the melting curve analysis. As a result of evaluating the analytic sensitivity of a primer kit of the present disclosure by setting a ratio of PIK3CA mutant DNA to target PIK3CA wild-type (WT) DNA to 0.001%, 0.01%, 0.1%, 1%, 10%, 50%, and 100%, mutations were detected from a mutation ratio of 0.001% (see FIGS. 1 and 4). Preferably, the mutations were detected from a mutation ratio of 0.01%. More preferably, it can be confirmed that the mutations were detected from a mutation ratio of 1% (see FIGS. 1 and 2 and FIGS. 4 and 5). In this regard, in previous research results, it was reported that mutations may be found as a result of sequencing only when the concentration of mutant DNA is 10% or higher, but compared to these previous reports, the present disclosure shows that the sensitivity of the kit of the present disclosure is very excellent by detecting mutations even at a mutant DNA ratio of 0.001%, which is much lower than 10%.

In the step of detecting the PIK3CA mutation in the companion animal by analyzing the gene amplification of the RT-qPCR, as a result of confirming the detection of the PIK3CA_E545K mutation using the composition for detecting the PIK3CA gene mutant tumor in the companion animal, a single melt peak was identified (see FIGS. 1 and 3), and it can be confirmed that since a region where fluorescence sensitivity decreased sharply was within the range of 81 to 84°C, there was a single specific melting temperature when confirming the melt curve (see FIGS. 1 and 3). It can be confirmed that the specificity of the PIK3CA_E545K mutation is very excellent using the composition for detecting the PIK3CA gene mutant tumor in the companion animal.

In the step of detecting the PIK3CA mutation in the companion animal by analyzing the gene amplification of the RT-qPCR, as a result of confirming the detection of the PIK3CA_H1047R, PIK3CA_H1047L or PIK3CA_H1047Y mutation using the composition for detecting the PIK3CA gene mutant tumor in the companion animal, a single melt peak was identified (see FIGS. 4 and 6), and it can be confirmed that since a region where fluorescence sensitivity decreased sharply was within the range of 81 to 84°C, there was a single specific melting temperature when confirming the melt curve (see FIGS. 4 and 6). It can be confirmed that the specificity of the PIK3CA_H1047R, PIK3CA_H1047L or PIK3CA_H1047Y mutation is very excellent using the composition for detecting the PIK3CA gene mutant tumor in the companion animal.

According to an embodiment of the present disclosure, the present disclosure relates to a method for providing information necessary for the treatment of a PIK3CA gene mutant tumor in a companion animal including: isolating a PIK3CA gene from a cancer sample or a sample suspected of having a cancer mutation; performing RT-qPCR by using the isolated PIK3CA gene as a template and using a primer set for detecting the PIK3CA gene mutant tumor in the companion animal including a forward primer selected from the group consisting of primers represented by SEQ ID NOs: 1 to 3 and a reverse primer selected from the group consisting of primers represented by SEQ ID NOs: 4 to 8; and identifying a specific site of the PIK3CA gene in the product amplified by the RT-qPCR.

The term 'sample' as used herein refers to a gene sample containing a gene mutation site to be detected. Specifically, all biologically derived samples capable of genetic analysis, including the nucleus and/or mitochondria, may be cells, tissues, organs, body fluids, etc., or endogenous or exogenous genes extracted therefrom.

In the step of isolating the PIK3CA gene from the cancer sample or the sample suspected of having the cancer mutation, the cancer sample or the sample suspected of having the cancer mutation may be collected from a blood sample of the companion animal. The companion animal may be a companion dog.

In the step of isolating the PIK3CA gene from the cancer sample or the sample suspected of having the cancer mutation, the isolating of the PIK3CA gene from the sample may be performed according to a general method known in the art.

In the step of performing the RT-qPCR by using the isolated PIK3CA gene as the template and using the primer set for detecting the PIK3CA gene mutant tumor in the companion animal including the forward primer selected from the group consisting of primers represented by SEQ ID NOs: 1 to 3 and the reverse primer selected from the group consisting of primers represented by SEQ ID NOs: 4 to 8, the RT-qPCR means a process of amplifying target DNA by using target DNA as a template and a primer specific to the target DNA, and repeating a process of denaturation, annealing, and extension reactions.

In the step of identifying the specific site of the PIK3CA gene of the product amplified by the RT-qPCR, the specific site of the PIK3CA gene means detecting at least one mutation selected from the group consisting of PIK3CA_E545K, PIK3CA_E542K, PIK3CA_H1047R, PIK3CA_H1047L, and PIK3CA_H1047Y.

According to an embodiment of the present disclosure, the present disclosure relates to a kit for use in detecting a PIK3CA gene mutant tumor in a companion animal, including at least one primer selected from the group consisting of base sequences represented by SEQ ID NOs: 1 to 8.

The RT-qPCR performed using the at least one primer selected from the group consisting of base sequences represented by SEQ ID NOs: 1 to 8 may rapidly identify the PIK3CA gene mutation in the companion animal with high reliability, thereby rapidly diagnosing a PIK3CA gene mutant tumor in a companion animal and rapidly providing information necessary for the treatment of the PIK3CA gene mutant tumor in the companion animal.

The term 'kit' used herein may include DNA polymerase, dNTPs, a buffer, etc. to perform the PCR amplification reaction. In addition, the kit may further include a user guideline describing optimal conditions for performing the reaction. The guideline is a printed document that describes a method for using a kit, for example, a method for preparing a reverse transcription buffer and a PCR buffer, presented reaction conditions, etc. The guideline includes pamphlets or leaflet-type brochures, labels attached to the kit, and instructions on the surface of a package including the kit. In addition, the guideline may include information published or provided through electronic media such as the Internet. The kit may have a shape such as a bottle, a tub, a sachet, an envelope, a tube, an ampoule, etc., which may be formed partially or entirely from plastic, glass, paper, foil, wax, etc. The container may be equipped with a cap that is initially a part of the container or may be attached to the container by mechanical, adhesive, or other means, and is fully or partially detachable. The container may also be equipped with a stopper that is accessible to the content by a syringe needle.

The detection of the PIK3CA gene mutant tumor in the companion animal may be used to evaluate the prognosis of cancer or predict the risk of developing a cancer mutation.

The detection of the PIK3CA gene mutant tumor in the companion animal may be used to provide information necessary for targeted cancer therapy.

Hereinafter, Examples will be described in detail to help the understanding of the present disclosure. However, the following Examples are merely illustrative of the content of the present disclosure, and the scope of the present disclosure is not limited to the following Examples. Examples of the present disclosure will be provided to more completely explain the present disclosure to those skilled in the art.

### Example 1

### 1. Template DNA

[Table 1] below shows PIK3CA gene information of a dog registered in the NCBI genebank to be used as a template for gene amplification in the present disclosure.

**[Table 1]**

| No | Source | Size (bp) | Accession Number |
|---|---|---|---|
| 1 | Canis lupus familiaris (dog) | 42263871 bp | NC_051838 |
| 2 | Canis lupus familiaris (dog) | 51113282 bp | NC_006616 |

### 2. Preparation of primer set

In order to design primers that produce a PCR product for a PIK3CA gene mutation, but do not produce a PCR product for a wild-type PIK3CA gene, eight primers were designed using the SnapGene program by confirming a primer size, a Tm value, a GC content of a primer, and whether there was a self-complementary sequence within the primer, etc. The base sequences of the designed primers were shown in [Table 2].

**[Table 2]**

| **No.** | **Name of Primer** | **Sequence (5'-3')** | **Length** | **Tm** | **%GC** |
|---|---|---|---|---|---|
| 1 | cPIK3CA-E545-WT-fwd-1 | gatcctctctctgaaatcactg | 22 | 54 | 45 |
| 2 | cPIK3CA-E545K-fwd-1 | gatcctctctctgaaatcactA | 22 | 53 | 41 |
| 3 | cfPIK3CA-H1047-fwd-2 | catacattcgaaagaccctagc | 22 | 55 | 45 |
| 4 | cfPIK3CA-E545-rve-1 | aggttagtaccaatgcagcgt | 21 | 59 | 48 |
| 5 | cPIK3CA-H1047-WT-rve-2 | tgttgtccagccaccatgatg | 21 | 60 | 52 |
| 6 | cPIK3CA-H1047R-rve-2 | ttgttgtccagccaccatgaG | 21 | 59 | 52 |
| 7 | cPIK3CA-H1047L-rve-2 | ttgttgtccagccaccatgaA | 21 | 59 | 48 |
| 8 | cPIK3CA-H1047Y-rve-2 | tgttgtccagccaccatgatA | 21 | 60 | 48 |

### Example 2

PCR of template DNA (Accession Number: NC_051838, NC_006616) was performed using the primers prepared in Example 1 above. Each 10 µL reaction contained 5 µL of a master mix, 1 µL of a primer (each with a final concentration of 0.1 µM), 2 µL of nuclease-free water, and 1 µL of template DNA (0.1 ng/µL). PCR performance was analyzed using Bio-Rad CFX Maestro software version 2.3. In all analyses, a threshold period (Ct) was determined by setting an automatic threshold. The PCR conditions were as shown in [Table 3].

**[Table 3]**

| | Temperature °C | Time | Cycles |
|---|---|---|---|
| Initial denaturation | 95 | 3 sec | |
| Denaturation | 95 | 10 sec | 40 |
| Annealing | 66 | 10 sec | |
| Extension | 72 | 30 sec | |
| Maintenance | 4 | Maintained | |

Through the experiment, it was confirmed that primers capable of targeting the PIK3CA gene mutations may selectively detect only DNA containing mutations without detecting normal DNA. Therefore, the primers can specifically detect tumor-inducing PIK3CA gene mutations and can be usefully used for the diagnosis of the PIK3CA gene mutant tumor in the companion animal.

### [Sequence list]

cPIK3CA-E545-WT-fwd-1: 5'- gatcctctctctgaaatcactg-3' (SEQ ID NO: 1)
cPIK3CA-E545K-fwd-1: 5'- gatcctctctctgaaatcacta -3' (SEQ ID NO: 2)
cfPIK3CA-H1047-fwd-2: 5'- catacattcgaaagaccctagc-3' (SEQ ID NO: 3)
cfPIK3CA-E545-rve-1: 5'- aggttagtaccaatgcagcgt-3' (SEQ ID NO: 4)
cPIK3CA-H1047-WT-rve-2: 5'- tgttgtccagccaccatgatg-3' (SEQ ID NO: 5)
cPIK3CA-H1047R-rve-2: 5'- ttgttgtccagccaccatgag-3' (SEQ ID NO: 6)
cPIK3CA-H1047L-rve-2: 5'- ttgttgtccagccaccatgaa-3' (SEQ ID NO: 7)
cPIK3CA-H1047Y-rve-2: 5'- tgttgtccagccaccatgata-3' (SEQ ID NO: 8)

## Claims

1. A primer set for detecting a PIK3CA gene mutant tumor in a companion animal comprising a forward primer selected from the group consisting of primers represented by SEQ ID NOs: 1 to 3 and a reverse primer selected from the group consisting of primers represented by SEQ ID NOs: 4 to 8.

2. The primer set for detecting the PIK3CA gene mutant tumor in the companion animal of claim 1, wherein the primer set for detecting the PIK3CA gene mutant tumor in the companion animal detects at least one mutation selected from the group consisting of PIK3CA_E545K, PIK3CA_E542K, PIK3CA_H1047R, PIK3CA_H1047L, and PIK3CA_H1047Y.

3. A composition for detecting a PIK3CA gene mutant tumor in a companion animal comprising at least any one primer set selected from the group consisting of:
a) a primer set consisting of a forward primer represented by SEQ ID NO: 1 and a reverse primer represented by SEQ ID NO: 4;
b) a primer set consisting of a forward primer represented by SEQ ID NO: 2 and a reverse primer represented by SEQ ID NO: 4;
c) a primer set consisting of a forward primer represented by SEQ ID NO: 3 and a reverse primer represented by SEQ ID NO: 5;
d) a primer set consisting of a forward primer represented by SEQ ID NO: 3 and a reverse primer represented by SEQ ID NO: 6;
e) a primer set consisting of a forward primer represented by SEQ ID NO: 3 and a reverse primer represented by SEQ ID NO: 7; and
f) a primer set consisting of a forward primer represented by SEQ ID NO: 3 and a reverse primer represented by SEQ ID NO: 8.

4. A composition for detecting a PIK3CA gene mutant tumor in a companion animal comprising at least any one primer set selected from the group consisting of:
a) a primer set consisting of a forward primer represented by SEQ ID NO: 1 and a reverse primer represented by SEQ ID NO: 4; and
b) a primer set consisting of a forward primer represented by SEQ ID NO: 2 and a reverse primer represented by SEQ ID NO: 4,
wherein the primer set detects a PIK3CA_E545K mutation.

5. A composition for detecting a PIK3CA gene mutant tumor in a companion animal comprising at least any one primer set selected from the group consisting of:
c) a primer set consisting of a forward primer represented by SEQ ID NO: 3 and a reverse primer represented by SEQ ID NO: 5;
d) a primer set consisting of a forward primer represented by SEQ ID NO: 3 and a reverse primer represented by SEQ ID NO: 6;
e) a primer set consisting of a forward primer represented by SEQ ID NO: 3 and a reverse primer represented by SEQ ID NO: 7; and
f) a primer set consisting of a forward primer represented by SEQ ID NO: 3 and a reverse primer represented by SEQ ID NO: 8,
wherein the primer set detects at least any one mutation selected from the group consisting of PIK3CA_H1047R, PIK3CA_H1047L and PIK3CA_H1047Y.

6. A method for diagnosing a PIK3CA gene mutant tumor in a companion animal comprising:
performing RT-qPCR for a PIK3CA gene in a companion animal using a primer set for detecting the PIK3CA gene mutant tumor in the companion animal including a forward primer selected from the group consisting of primers represented by SEQ ID NOs: 1 to 3 and a reverse primer selected from the group consisting of primers represented by SEQ ID NOs: 4 to 8; and
detecting a PIK3CA mutation in the companion animal by analyzing gene amplification of the RT-qPCR.

7. The method for diagnosing the PIK3CA gene mutant tumor of claim 6, wherein the PIK3CA gene mutant tumor in the companion animal is a mammary tumor.

8. A method for providing information necessary for treatment of a PIK3CA gene mutant tumor in a companion animal, comprising:
isolating a PIK3CA gene from a cancer sample or a sample suspected of having a cancer mutation;
performing RT-qPCR by using the isolated PIK3CA gene as a template and using a primer set for detecting the PIK3CA gene mutant tumor in the companion animal including a forward primer selected from the group consisting of primers represented by SEQ ID NOs: 1 to 3 and a reverse primer selected from the group consisting of primers represented by SEQ ID NOs: 4 to 8; and
identifying a specific site of the PIK3CA gene in a product amplified by the RT-qPCR.

9. The method for providing information necessary for treatment of the PIK3CA gene mutant tumor in the companion animal of claim 8, wherein the cancer sample or the sample suspected of having the cancer mutation is collected from a blood sample of the companion animal.

10. A kit for use in detecting a PIK3CA gene mutant tumor in a companion animal, comprising at least one primer selected from the group consisting of base sequences represented by SEQ ID NOs: 1 to 8.
